# EUROPÄISCHE PATENTANMELDUNG

(11) **EP 4 016 070 A1**
(43) Veröffentlichungstag der Anmeldung: **22.06.2022**
(21) Anmeldenummer: 21212138.8
(22) Anmeldetag: 03.12.2021
(51) Int. Cl.: G01N 27/404

(54) **ELEKTROCHEMISCHER GASSENSOR**

(30) Priorität: 21.12.2020 DE 102020134465
(71) Anmelder: Dräger Safety AG & Co. KGaA, 23560 Lübeck (DE)
(72) Erfinder: Mett, Frank, 23558 Lübeck (DE)
(74) Vertreter: Guthöhrlein, Gerhard

(57) **Zusammenfassung**

Die vorliegende Erfindung betrifft einen elektrochemischen Gassensor für saure Analytgase, wobei der elektrochemische Gassensor ein Absorptionsmittel aufweist, welches dazu geeignet ist ein an der Elektrode entstehendes Reaktionsprodukt zu absorbieren, und wobei der elektrochemische Gassensor weiterhin eine Borverbindung aufweist, welche dazu geeignet ist, mit dem sauren Analytgas chemisch zu reagieren. Weiterhin betrifft die vorliegende Erfindung ein Verfahren zur Konzentrationsbestimmung saurer Gase sowie die Verwendung eines elektrochemischen Gassensors zur Konzentrationsbestimmung von sauren Gasen.

## Beschreibung

Die vorliegende Erfindung betrifft einen elektrochemischen Gassensor mit einem Absorptionsmittel und einer Borverbindung, welcher eine verbesserte Langzeitsignalstabilität gegenüber aus dem Stand der Technik bekannten Gassensoren bei Beaufschlagung mit sauren Analytgasen aufweist. Weiter betrifft die Erfindung die Verwendung eines erfindungsgemäßen Gassensors zur Messung von sauren Analytgasen, insbesondere zur Konzentrationsbestimmung von Fluorwasserstoff sowie ein Verfahren zur Konzentrationsmessung von sauren Analytgasen mit einem elektrochemischen Gassensor.

Elektrochemische Gassensoren sind aus dem Stand der Technik allgemein bekannt. Üblicherweise weisen derlei Gassensoren eine Arbeitselektrode (auch Messelektrode genannt), eine Gegenelektrode (auch Hilfselektrode genannt) und optional eine Referenzelektrode und eine Schutzelektrode auf. Diese Elektroden werden zumeist von einem Sensorgehäuse umschlossen und stehen in Kontakt mit einem in der Regel flüssigen Elektrolyten. Arbeits- und Gegenelektrode sind üblicherweise durch Separatoren getrennt, die elektrolytdurchlässig und gasundurchlässig sind. Die Arbeits- und Gegenelektrode stehen in leitenden Kontakt mit dem Elektrolyten und bilden so ein galvanisches Element (elektrochemische Messzelle).

Elektrochemische Gassensoren werden in einer Vielzahl von Anwendungen eingesetzt, bspw. zur Detektion toxischer Gase in Industrieanlagen, in Produktionsstätten, an Pipelines, in Materiallagern bzw. bei der Prozessüberwachung in der chemischen Industrie und werden unter anderem als stationäre oder auch als portable Gassensoren eingesetzt. Die elektrochemischen Gassensoren werden insbesondere dafür eingesetzt, kritische Konzentration von giftigen und/oder explosiven Gasen frühzeitig zu erkennen, um rechtzeitig vor einer bevorstehenden Gefahr zu warnen. Weiter werden elektrochemische Gassensoren eingesetzt, um saure Gase, d.h. Gase, welche in wässriger Lösung einen pH-Wert von kleiner 7 aufweisen, wie sie unter anderem bei der Aluminiumproduktion, bei der Uranaufbereitung, in der Halbleiterindustrie bzw. bei der Herstellung von Halbleitern oder auch beim Brand von Elektrofahrzeugen, entstehen, zu erkennen bzw. zu messen. Unter sauren Gasen werden allgemein solche Gase verstanden, welche beim Lösen bzw. Einleiten in Wasser einen pH-Wert kleiner 7 aufweisen. Hierzu zählen unter anderem Fluorwasserstoff (Flusssäure; HF), Chlorwasserstoff (Salzsäure; HCl), Bromwasserstoff (HBr) oder auch Schwefelwasserstoff (H₂S) und Schwefeldioxid (SO₂).

GB 2129562 B offenbart den elektrochemischen Nachweis von Fluorwasserstoff (HF), bei denen Platindrähte als Elektroden eingesetzt werden. Als Elektrolyt wird ein Gemisch aus Calciumbromid und Calciumbromat verwendet. Nachteilig ist jedoch besonders die Temperaturabhängigkeit der Nachweismethode.

Aus WO 2002/031485 A1 ist bekannt eine Messelektrode aus einem elektrochemisch aktiven Metalloxidpulver einzusetzen. Solche Metalloxidpulver können jedoch hohe Querempfindlichkeiten auf andere Gase aufweisen, wodurch sich geringe Konzentrationen des Zielgases nicht immer sicher nachweisen lassen.

WO 1999/001758 A1 offenbart einen elektrochemischen Sensor, der insbesondere zum Nachweis von HCl geeignet ist. Bei diesem Sensor weist die Arbeitselektrode eine elektrochemisch aktive Oberfläche aus Gold auf. Diese löst sich jedoch mit der Zeit auf, was im Ernstfall zum Ausfall des Sensors führen kann.

DE 102014002500 A1 offenbart einen elektrochemischen Gassensor für saure Gase mit einem Absorptionsmittel, welches dazu geeignet ist, mit einem Reaktionsprodukt zu reagieren. Als mögliches Absorptionsmittel ist insbesondere Bariumcarbonat (BaCO₃) offenbart, welches mit HF zu Bariumfluorid und Kohlensäure gemäß folgender Reaktionsgleichung reagieren kann: 2 HF + BaCO₃ → BaF₂ + H₂CO₃. Nachteilig bei dem vorgeschlagenen Sensor ist jedoch, dass die Langzeitsignalstabilität beeinträchtigt wird, wenn dieser zur Messung bzw. Erkennung von sauren Gasen, insbesondere von HF eingesetzt wird. Dies macht sich unter anderem dadurch bemerkbar, dass nach einer gewissen Einsatzzeit bei einem Gaswechsel von Luft auf ein HF-haltiges Prüfgas bzw. Analytgas die Messsignale des vorgeschlagenen elektrochemischen Gassensors auf einen geringen Wert einbrechende Signale aufweisen, welche das Messergebnis verfälschen bzw. nicht richtig wiedergeben. Dies ist jedoch unerwünscht und beeinträchtigt das Messergebnis und somit auch die Kosten für Wartung und Erneuerung des Sensors.

Es besteht somit nach wie vor ein Bedarf an elektrochemischen Gassensoren, welche insbesondere für die Messung bzw. zum Nachweis von sauren Gasen eingesetzt werden können, und welche eine hohe Langzeitsignalstabilität d. h. ein zuverlässiges Messergebnis über einen langen Zeitraum bereitstellen können, ohne dass das Messergebnis nach einer gewissen Einsatzzeit auf geringe Messwerte "einbricht", das heißt verfälscht wird.

Aufgabe der vorliegenden Erfindung ist es somit, einen elektrochemischen Gassensor bereitzustellen, welcher die aus dem Stand der Technik bekannten Probleme umgeht. Es soll somit ein elektrochemischer Gassensor bereitgestellt werden, welcher sich für die Messung bzw. Erkennung von sauren Gasen, wie beispielsweise Fluorwasserstoff (HF), Chlorwasserstoff (HCl), Bromwasserstoff (HBr), lodwasserstoff (HI), Schwefelwasserstoff (H₂S), Schwefeldioxid (SO₂) und Essigsäure (AcOH) und anderen eignet, der langzeitstabil ist, ohne dass die Messsignale nach einem gewissen Zeitpunkt "einbrechen" bzw. ungenau werden, und der eine hohe Dauerbegasungsfestigkeit, d.h. eine hohe Langzeitsignalstabilität idealerweise auch über einen Zeitraum von wenigstens 12 bis 18 Monaten aufweist. Darüber hinaus ist es Aufgabe der vorliegenden Erfindung einen elektrochemischen Gassensor bereitzustellen, welcher kostengünstig vom Endabnehmer betrieben werden kann, ohne dass dieser eine hohe Wartungsintensität bzw. eine Vielzahl an Wartungszyklen benötigt.

Eine weitere Aufgabe der vorliegenden Erfindung ist es ein Verfahren zur Messung bzw. zur Konzentrationsbestimmung von sauren Gasen bereitzustellen, mit welcher eine höhere Genauigkeit insbesondere über einen Zeitraum von wenigstens 12 bis 18 Monaten durchgeführt werden kann, ohne dass ein sog. Signaldrift auftritt, was zu einer Verfälschung der Messergebnisse führt.

Diese und weitere Aufgaben werden erfindungsgemäß durch den Inhalt der unabhängigen Patentansprüche gelöst. Bevorzugte Ausgestaltungen sind Inhalt der Unteransprüche bzw. nachfolgend genauer beschrieben.

Überraschenderweise wurde gefunden, dass bei gleichzeitiger Verwendung einer Borverbindung neben einem Absorptionsmittel die Langzeitsignalstabilität und damit die Messgenauigkeit eines elektrochemischen Gassensors für saure Gase, d.h. für Gase, welche in wässriger Lösung einen pH-Wert von kleiner 7 aufweisen, über einen langen Begasungszeitraum deutlich verbessert werden kann.

Erfindungsgemäß wird ein elektrochemischer Gassensor vorgeschlagen, der ein Gehäuse mit darin angeordneter Arbeitselektrode und Gegenelektrode, einen mit Arbeits- und Gegenelektrode in leitendem Kontakt stehenden Elektrolyten sowie ein Absorptionsmittel aufweist, wobei das Absorptionsmittel dazu geeignet ist, ein an der Arbeitselektrode bzw. ein an der Gegenelektrode entstehendes Reaktionsprodukt zu absorbieren. Weiterhin weist der erfindungsgemäße elektrochemische Gassensor eine Borverbindung auf, welche dazu geeignet ist, mit einem den Gassensor durchlaufenden sauren Analytgas chemisch zu reagieren.

Weiterhin wird ein Verfahren zur Konzentrationsbestimmung saurer Analytgase vorgeschlagen, mit welchem über einen langen Zeitraum eine hohe Langzeitsignalstabilität gewährleistet wird. Das Verfahren umfasst dabei die Beaufschlagung eines Gassensors mit dem sauren Analytgas, welches an der Arbeitselektrode reduziert wird und die migrierenden Reaktionsprodukte an der Gegenelektrode oxidiert werden. Das saure Analytgas reagiert chemisch mit einer im Gassensor angeordneten Borverbindung und die an der Gegenelektrode entstehenden Reaktionsprodukte werden durch ein Absorptionsmittel absorbiert.

Weiterhin ist die Verwendung eines elektrochemischen Gassensor für die Messung bzw. Konzentrationsbestimmung von sauren Gasen, insbesondere halogenhaltigen Gasen wie HF, Teil der vorliegenden Erfindung.

Das Analytgas ist das Gas, mit dem der elektrochemische Gassensor beaufschlagt wird. Das Analytgas weist in wässriger Lösung einen pH-Wert von kleiner 7 auf, und wird im Sinne der vorliegenden Erfindung auch als saures Gas bezeichnet. Üblicherweise diffundiert das Analytgas zusammen mit Luft aus der Umgebungsatmosphäre an den erfindungsgemäßen Gassensor, bzw. der erfindungsgemäße elektrochemische Gassensor wird damit beaufschlagt. Das Analytgas ist in wässriger Lösung ein saures Gas bzw. kann auch saure Dämpfe, wie beispielsweise Essigsäure umfassen oder beides und umfasst vorliegend Halogenwasserstoffe und/oder Schwefelwasserstoff oder Schwefeldioxid, d.h. das Analytgas kann beispielsweise Halogen- und/oder Schwefel-und/oder Wasserstoffatome aufweisen. Vorzugsweise ist das Analytgas ein Halogenwasserstoff, wie bspw. Fluorwasserstoff (HF), Chlorwasserstoff (HCl), Bromwasserstoff (HBr), lodwasserstoff (HI) oder aber Schwefelwasserstoff (H₂S), Schwefeldioxid (SO₂) und Essigsäure (AcOH) bzw. Mischungen davon, bevorzugt Halogenwasserstoffe wie HF, HCl, HBr und/oder HI oder Mischungen, besonders bevorzugt HF. Typischerweise ist das Analytgas in der Umgebungsatmosphäre des Gassensors umfasst bzw. hierin beinhaltet, kann aber auch dem Gassensor gesondert, z.B. in wohl definierten Dosen, batchweise zugeführt werden.

Das Gehäuse des elektrochemischen Gassensors begrenzt diesen nach außen. Üblicherweise steht das Gehäuse nach außen hin mit der Umgebungsatmosphäre in Kontakt und bildet im Inneren des Gehäuses eine Aufnahme für die Elektroden wie die Arbeitselektrode, die Gegenelektrode und optional eine Referenzelektrode und/oder eine Schutzelektrode sowie für den Elektrolyten. Das Gehäuse kann aus unterschiedlichen, für den Einsatz von elektrochemischen Gassensoren typischen Materialien hergestellt werden. Hierzu zählen beispielsweise Kunststoffe wie bspw. Polyethylen (PE), Polypropylen (PP), Perfluoralkoxy-Polymere (PFA), Fluorethylenpropylen (FEP) oder Polytetrafluorethylen (Teflon; PTFE). Das Gehäuse des elektrochemischen Gassensors weist weiterhin ein Elektrolytreservoir für die Aufnahme des Elektrolyten auf.

Bei dem erfindungsgemäßen Gassensor diffundiert in einer Ausgestaltung das Analytgas aus der Umgebungsluft durch eine hydrophobe (wasserabweisende) Membran in den elektrochemischen Gassensor und gelangt an die Dreiphasengrenze, welche von der Arbeitselektrode, dem Elektrolyten sowie dem Analytgas gebildet wird. Das Analytgas reagiert schließlich an der Arbeitselektrode (Hinreaktion), wobei die sich an der Gegenelektrode bildenden Reaktionsprodukte (Rückreaktion) schließlich wieder in den Elektrolyten gelangen und wieder aus dem elektrochemischen Gassensor hinausströmen. Sensoren, welche analog dieses Prinzips arbeiten (Reduktion an der Arbeitselektrode und Oxidation an der Gegenelektrode), werden umgangssprachlich auch als Pump-Sensoren bezeichnet. Pump-Sensoren werden zumeist für den Nachweis von Sauerstoff (O₂), Ammoniak und oder Halogenwasserstoffen eingesetzt.

Andere aus dem Stand der Technik bekannte elektrochemische Gassensoren funktionieren so, dass das in den Gassensor diffundierende Analytgas an der Arbeitselektrode oxidiert wird (zum Beispiel Kohlenmonoxid zu Kohlendioxid oder Schwefelwasserstoff zu Schwefelsäure) und an der Gegenelektrode wiederum Sauerstoff reduziert wird.

Im einfachsten Fall weist das Gehäuse eine Öffnung zum Gasaustausch mit der Umgebung und einen elektrolytgefüllten Reaktionsraum auf, in welchem die Elektroden angeordnet sind. In dieser Ausgestaltung diffundiert das Analytgas beispielsweise durch die Öffnung und optional eine Messelektrodenmembran in den Reaktionsraum des Gassensors hinein und weiter an die Arbeitselektrode. An der Arbeitselektrode findet eine Hinreaktion stattfindet. In einer weiteren Ausgestaltung ist es auch möglich, dass das Gehäuse sowohl einen Gaseinlass als auch einen Gasauslass aufweist, wobei der Diffusionsweg vom Gaseinlass zur Arbeitselektrode und von der Gegenelektrode zum Gasaustausch führt. In dieser Ausgestaltung ist es möglich, dass das an der Gegenelektrode gebildete Gas durch den Elektrolyten und optional eine Membran über den Gasauslass aus dem Gassensor diffundiert.

Das Absorptionsmittel der vorliegenden Erfindung ist derart ausgebildet, dass es mit wenigstens einem an der Gegenelektrode und/oder an der Arbeitselektrode entstehenden Reaktionsprodukt reagieren kann, vorzugsweise mit einem an der Gegenelektrode entstehenden Reaktionsprodukt. Das Absorptionsmittel kann noch weitere Inhaltsstoffe bzw. Zusatzstoffe und/oder Bindemittel, wie zum Beispiel Teflon (Polytetrafluorethylen; PTFE), Cellulose, Baumwolle und/oder Polymerfasern umfassen, welche insbesondere dazu geeignet sind, das Absorptionsmittel innerhalb des Gehäuses des elektrochemischen Gassensors und/oder Nahe der Elektroden, insbesondere der Gegenelektrode, anzuordnen. So kann das Absorptionsmittel beispielsweise auf ein Polymervlies oder auf ein Glasvlies geträgert sein oder auch auf der Arbeits- und/oder der Gegenelektrode angeordnet sein. Bevorzugt ist das Absorptionsmittel an der Gegenelektrode angeordnet bzw. auf dieser angebracht, so dass die an der Gegenelektrode entstehenden Reaktionsprodukte auf diese Weise direkt an der Gegenelektrode absorbiert bzw. abgefangen werden können. In einer bevorzugten Ausgestaltung kann das Absorptionsmittel Teflon umfassen.

In einer weiter bevorzugten Ausgestaltung umfasst das Absorptionsmittel zusätzlich Pulpe. Dabei handelt es sich um eine Suspension, welche beispielsweise Wasser und Glasfasern umfassen, welches kostengünstig hergestellt werden kann und insbesondere formbar und formstabil ist. Alternativ können auch Suspensionen aus Cellulose, Baumwolle und/oder Polymerfasern verwendet werden. In einigen Ausgestaltungen können der Pulpe zusätzlich Zuschlagsstoffe wie Ton oder Kreide zugesetzt werden. Pulpe ist insbesondere dazu geeignet, mit dem Absorptionsmittel vermengt zu werden, da die Pulpe das Absorptionsmittel, welches häufig nicht formstabil bzw. nicht oder nur schwer formbar ist, da dies amorph und/oder kristallin oder flüssig vorliegt, gewissermaßen formbar und formstabil macht beziehungsweise sich das Absorptionsmittel dadurch gewissermaßen formen lässt. So ist es insbesondere möglich, dass Absorptionsmittel mit einer wässrigen Pulpe zu vermengen, in eine vorgegebene Form einzubringen, und in dieser Form ein elektrisch leitfähiges Elektrodenmaterial, wie beispielsweise Kohlenstofffasern, Kohlenstoffnanoröhren (engl. Carbon nanotubes = CNT), Graphen, Graphenoxid, Glaskohlenstoff, reduziertes Graphenoxid (engl. reduced graphene oxide = rGO), diamantähnlicher Kohlenstoff (engl. diamond-like carbon = DLC) einzuarbeiten. Dies ist insbesondere bevorzugt, da auf diese Weise das Absorptionsmittel direkt auf die Elektrode, bevorzugt auf die Gegenelektrode, geträgert bzw. angeordnet werden kann. Das Absorptionsmittel, die Glasfasern der Pulpe und das Elektrodenmaterial, bevorzugt Kohlenstoffnanoröhren, ergeben nach Trocknung ein Verbundmaterial, welches bevorzugt als Elektrode eingesetzt wird. In einer weiteren Ausgestaltung ist es auch möglich, dass Absorptionsmittel direkt auf das Elektrodenmaterial zu trägem.

Beim Einsatz von HF als Analytgas entstehen an der Arbeitselektrode zunächst FluoridAnionen (Reduktion), welche anschließend an der Gegenelektrode wieder zu HF oxidiert werden. Eine Freisetzung von HF aus dem Gassensor ist jedoch in der Regel nicht gewünscht da dieses u.a. hochgiftig und ätzend ist. Eine Ansammlung bzw. Anreicherung von HF innerhalb des elektrochemischen Gassensors ist ebenfalls unerwünscht, da eine Ansammlung von HF im Inneren des Sensors zu einer sogenannten Sensordrift führen kann, was zur Folge hat, dass die Messergebnisse ungenau werden bzw. diese verfälscht werden. Deshalb ist es vorteilhaft, wenn das an der Gegenelektrode gebildete HF mit dem Absorptionsmittel reagieren kann. In einer bevorzugten Ausgestaltung ist das Absorptionsmittel so gewählt, dass das an der Gegenelektrode entstehende HF in einen schwerlöslichen, bzw. im Elektrolyten ausfallenden Feststoff umgesetzt wird.

Auch ist es möglich, dass das Absorptionsmittel an einer Stelle des Gehäuses oder an mehreren Stellen des Gehäuses des elektrochemischen Gassensors angeordnet ist oder sind. So wird in einer Ausgestaltung des Absorptionsmittel auf ein Polymervlies geträgert und das so geträgerte Polymervlies anschließend innerhalb des Gassensors angeordnet. Bevorzugt wird das Absorptionsmittel, wie bereits zuvor beschrieben, dabei in der Nähe der Arbeitselektrode bzw. der Gegenelektrode angeordnet, insbesondere bevorzugt in der Nähe der Gegenelektrode.

In einer weiteren Ausgestaltung ist es möglich, dass das Absorptionsmittel als Stopfen im Gasauslass angeordnet ist. So kann das Absorptionsmittel einen Filter bilden, durch welchen das aus dem Gassensor ausströmende Gas hindurchströmen bzw. hindurch diffundieren muss. Dabei kann dann das Absorptionsmittel mit dem an der Arbeitselektrode oder an der Gegenelektrode, insbesondere an der Gegenelektrode entstehenden Reaktionsprodukt reagieren, so dass eine Freisetzung an die Umwelt effektiv verhinderbar ist. Gleichzeitig kann das Absorptionsmittel dabei eine Außenbegrenzung des elektrolytgefüllten Reaktionsraums bilden. Es ist bevorzugt das Absorptionsmittel so zu wählen, dass eine mit dem Reaktionsprodukt an der Gegenelektrode entstehende Verbindung entsteht, welche insbesondere im Elektrolyten schwer löslich ist bzw. im Elektrolyten ausfällt.

In einer bevorzugten Ausgestaltung umfasst das Absorptionsmittel eine Carbonatverbindung bzw. besteht aus dieser, insbesondere Alkalicarbonate und/oder Erdalkalicarbonate, bevorzugt ist Calciumcarbonat und/oder Bariumcarbonat, insbesondere bevorzugt Bariumcarbonat. Dabei kann an der Gegenelektrode entstehendes HF nach folgender Reaktionsgleichung absorbiert werden: 2 HF + BaCO₃ → BaF₂ + H₂CO₃. Bariumfluorid ist schwerlöslich und fällt im Elektrolyten aus. Das ausfallende Bariumfluorid lagert sich zwar an der Gegenelektrode an, jedoch ohne die Gegenelektrode zu vergiften (d. h. ohne die Oberfläche der Elektrode zuzusetzen und damit für weitere Reaktion zu versiegeln).

Zum Nachweis von sauren Gasen (welche in wässriger Lösung einen pH-Wert kleiner 7 aufweisen), insbesondere Halogenwasserstoffgasen ist es vorteilhaft, wenn der Elektrolyt eine Zusammensetzung ist, die ein organisches Lösungsmittel und ein Leitsalz enthält, bevorzugt eine Zusammensetzung, die ein organisches Lösungsmittel, welches ein chinoides System enthält, bspw. aus der Gruppe der Chinone, und ein Leitsalz, welches ein organisches Kation aufweist, enthält.

Das Leitsalz kann beispielsweise eine ionische Flüssigkeit sein. Bevorzugt ist das Anion des Leitsalzes dabei ausgewählt aus der Gruppe enthaltend Halogenid, Carbonat, Sulfonat, Phosphat und/oder Phosphonat, bevorzugt ein Anion ausgewählt aus der Gruppe enthaltend Alkyl-sulfonat, Alkenyl-Sulfonat, Aryl-Sulfonat, Alkyl-phosphat, Alkenyl-Phosphat, Aryl-Phosphat, substituiertes Alkyl-sulfonat, substituiertes Alkenyl-sulfonat, substituiertes Aryl-sulfonat, substituiertes Alkyl-phosphat, substituiertes Alkenyl-phosphat, substituiertes Aryl-phosphat, halogeniertes Phosphat, halogeniertes Sulfonat halogeniertes Alkyl-sulfonat, halogeniertes Alkenyl-sulfonat, halogeniertes Aryl-sulfonat, halogeniertes Alkyl-phosphat, halogeniertes Alkenyl-phosphat, halogeniertes Aryl-phosphat, besonders bevorzugt ein Anion ausgewählt aus der Gruppe enthaltend Fluorophosphat, Alkylfluorophosphat, Aryl-sulfonat, ganz besonders bevorzugt aus der Gruppe enthaltend Perfluoralkylfluorophosphat, Toluolsulfonat.

Dabei ist es vorteilhaft, wenn das Leitsalz als Kationen Metallionen, Oniumionen oder ein Gemisch aus Metallionen und Oniumionen enthält. Zum Beispiel können die Metallionen ausgewählt sein aus Alkalimetallionen oder Erdalkalimetallionen, bevorzugt aus Li, K und/oder Na. Günstig ist es, wenn die Oniumionen ausgewählt sind aus Ammonium-, Phosphonium, Guanidiniumkationen und heterocyclischen Kationen, bevorzugt ausgewählt aus Alkyl-Ammonium- und heterocyclischen Kationen, besonders bevorzugt ausgewählt aus Alkyl-Ammonium, Imidazolium und/oder substituierten Imidazoliumionen, wobei die substituierten Imidazolium-Ionen bevorzugt eine Struktur entsprechend 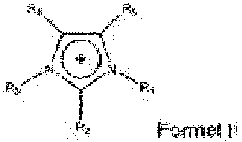 aufweisen, wobei R1, R2, R3, R4 und R5 unabhängig voneinander ausgewählt sein können aus -H, geradkettiges oder verzweigtes Alkyl mit 1 bis 20 C-Atomen, geradkettiges oder verzweigtes Alkenyl mit 2 bis 20 C-Atomen und einer oder mehreren Doppelbindungen, geradkettiges oder verzweigtes Alkinyl mit 2 bis 20 C-Atomen und einer oder mehreren Dreifachbindungen, gesättigtes, teilweise oder vollständig ungesättigtes Cycloalkyl mit 3-7 C-Atomen, das mit Alkylgruppen mit 1 bis 6 C-Atomen substituiert sein kann, gesättigtes teilweise oder vollständig ungesättigtes Heteroaryl, Heteroaryl-C1-C6-alkyl oder Aryl-C1-C6-alkyl, wobei besonders bevorzugt R2, R4 und R5 H sind und R1 und R3 jeweils unabhängig voneinander ein geradkettiges oder verzweigtes Alkyl mit 1 bis 20 C-Atomen.

### Beispielsweise ist insbesondere vorstellbar, dass als Leitsalz

Tetrabutlyammoniumtoluolsulfonat oder 1-Hexyl-3-methyl-imidazolium-tris(pentafluoroethyl)-trifluorophosphat verwendet wird. So ist es insbesondere vorteilhaft, wenn der Elektrolyt ein Gemisch aus einem Lösungsmittel, einem Leitsalz und/oder einem organischen Mediator ist, Alkylammonium-toluolsulfonat und ionischen Flüssigkeiten, mit einem Perfluoralkylfluorophosphat-Anion.

Weiterhin ist es günstig, wenn der organische Mediator eine Polyhydroxyverbindung ist, welche bei Oxidation ein chinoides System oder ein Naphthalin-System bildet. Beispielsweise kann der organische Mediator ausgewählt sein aus der Gruppe enthaltend ortho-Dihydroxybenzol, para-Dihydroxybenzol, substituierte ortho-Dihydroxybenzole und substituierte para-Dihydroxybenzole, Dihydroxynaphtalin, substituiertes Dihydroxynaphtalin, Anthrahydrochinon, substituiertes Anthrahydrochinon, bevorzugt 1,2-Dihydroxybenzol, 1,4 Dihydroxybenzol, Naphtohydrochinon, substituiertes 1,2- oder 1,4.Dihydroxybenzol, substituiertes Hydrochinon, substituiertes Naphtohydrochinon, substituiertes Anthrahydrochinon, besonders bevorzugt substituiertes Hydrochinon, substituiertes 1,2-Dihydroxybenzol. Dabei ist es besonders günstig, wenn die Substituenten des substituierten Anthrachinons, substituierten 1,2-Dihydroxybenzol und/oder substituierten 1,4-Hydrochinon ausgewählt sind aus der Gruppe enthaltend Sulfonyl, tert-Butyl, Hydroxy, Alkyl, Aryl, bevorzugt und/oder tert-Butyl.

Die Borverbindung der vorliegenden Erfindung ist so gewählt, dass diese chemisch mit dem Analytgas reagieren kann. Borverbindung im Sinne der vorliegenden Erfindung meint eine Verbindung, welche zumindest ein Boratom enthält. Hierzu zählen sämtliche Borverbindungen, welche mit sauren Gasen, d. h. mit Gasen, welche in Wasser gelöst einen pH-Wert kleiner 7 aufweisen, chemisch reagieren können. Insbesondere handelt es sich bei der Borverbindung des erfindungsgemäßen elektrochemischen Gassensors um Borsäure (H₃BO₃) und/oder Bortrioxid (B₂O₃). Die Borverbindung ist im Gehäuse des erfindungsgemäßen elektrochemischen Gassensors angeordnet. In einer bevorzugten Ausgestaltung ist die Borverbindung in einer Trennschicht zwischen der Arbeitselektrode und der Gegenelektrode angeordnet. Üblicherweise werden Arbeitselektrode und Gegenelektrode mittels einer Separatorschicht (Trennschicht) bzw. mittels eines Separatormaterials zur Vermeidung von elektrischen Kurzschlüssen voneinander getrennt. Als Separatormaterial können sämtliche, aus dem Stand der Technik bekannten Materialien eingesetzt werden. Bevorzugt werden Glasvliese und oder Polymervliese erfindungsgemäß eingesetzt, welche semipermeabel sind. Es ist auch möglich, elektrolytundurchlässige Trennschichten einzusetzen, sogenannte semipermeable Membranen.

Die Borverbindung kann unter anderem in Pulver- oder Tablettenform vorliegen. In einer anderen Ausgestaltung wird die Borverbindung als Paste im Gassensor angeordnet. In noch einer weiteren Ausgestaltung liegt die Borverbindung als Borosilikatglas vor. Borosilikatglas umfasst unter anderem Siliziumdioxid, Bortrioxid, Alkalioxide, Erdalkalioxide sowie Aluminiumoxid. In der bevorzugten Ausgestaltung ist die Borverbindung auf ein Vlies geträgert. Dazu können unter anderem Kunststoffvliese und/oder Glasvliese verwendet werden. So ist es möglich, die Borverbindung in einem geeigneten Lösungsmittel zu lösen und anschließend ein Polymervlies und/oder ein Glasvlies damit zu tränken. Ein so mit der Borverbindung getränktes Polymervlies bzw. Glasvlies kann direkt in dem elektrochemischen Gassensor eingesetzt werden. In einer weiteren Ausgestaltung wird das getränkte Vlies zunächst getrocknet und anschließend im Gassensor angeordnet, insbesondere zwischen Arbeits- und Gegenelektrode. Bevorzugt werden als Polymervliese solche eingesetzt, die aus Polypropylen und/oder Polyethylen hergestellt sind, insbesondere solche aus Polypropylen.

Die Borverbindung kann an prinzipiell an unterschiedlichen Stellen innerhalb des Gehäuses angeordnet sein, bevorzugt ist diese zwischen der Arbeitselektrode und der Gegenelektrode angeordnet. Insbesondere als vorteilhaft hatte sich herausgestellt, wenn die Borverbindung sich in einer Trennschicht befindet bzw. auf diese aufgeträgert wird, und diese Trennschicht zwischen der Arbeitselektrode und der Gegenelektrode angeordnet wird. Die Trennschicht umfasst insbesondere Vliese, wie Glasvliese und/oder Kunststoffvliese bzw. besteht die Trennschicht aus den genannten Vliesen, welche zumindest teilweise mit der Borverbindung geträgert sind.

Ohne an die Theorie gebunden zu sein vermuten die Erfinder, dass sich der gegenüber den aus dem Stand der Technik bekannten Gassensoren erzielte Signalverbesserungseffekt bzw. die verbesserte Langzeitsignalstabilität dadurch ergibt, dass das Analytgas, in diesem Falle Fluorwasserstoff, mit Borsäure reagiert, so dass die an der Arbeitselektrode entstehenden Ionen verbessert zur Gegenelektrode wandern können gemäß folgernder Reaktionsgleichung: 4 HF + H₃BO₃ → [H]⁺ [BF₄]⁻ + 3 H₂O Arbeitselektrode und Gegenelektrode können aus gleichen oder unterschiedlichen, dem Fachmann bekannten Materialen hergestellt sein, beispielsweise aus Kohlenstofffasern bzw. aus Edelmetallen, wie Gold, Platin und/oder Iridium. Die Arbeitselektrode umfasst bevorzugt elektrisch leitfähigen Kohlenstofffasern, besonders bevorzugt Kohlenstoffnanoröhren (CNT), Graphen und/oder diamantähnlichem Kohlenstoff (engl. diamond-like carbon = DLC) und insbesondere bevorzugt umfasst die Arbeitselektrode reduziertes Graphenoxid (engl. reduced graphene oxide = rGO).

Die Gegenelektrode umfasst bevorzugt Kohlenstofffasern, besonders bevorzugt Graphen und/oder Glaskohlenstoff und insbesondere bevorzugt Kohlenstoffnanoröhren (CNT).

Es ist bevorzugt, das Absorptionsmittel an der Gegenelektrode anzuordnen. Dabei ist es insbesondere bevorzugt, Kohlenstoffnanoröhren in einer wässrigen Pulpe anzuordnen, wobei die wässrige Pulpe mit einem oder mehreren Absorptionsmitteln vermengt wird. Nach dem Trocknen der wässrigen Pulpe ergibt sich so ein Verbundmaterial umfassend Kohlenstoffnanoröhren als Elektrodenmaterial, Absorptionsmittel und Glasfasern. Dies ist insbesondere vorteilhaft, da auf diese Weise das Absorptionsmittel direkt auf die Gegenelektrode geträgert bzw. angeordnet wird, um dort das entstehende Reaktionsprodukt wie z.B. HF zu absorbieren und so "einzufangen", das heißt, dass an der Gegenelektrode entstehende HF wird nicht in die Umgebungsatmosphäre abgegeben. In einer weiteren Ausgestaltung wird ein offenporiger Kohlenstoffschwamm als Gegenelektrode eingesetzt. In dieser Ausgestaltung ist es vorteilhaft, wenn das Absorptionsmittel wenigstens teilweise in den Poren des Kohlenstoffschwamms eingebracht ist.

In einer besonders bevorzugten Ausgestaltung umfasst ein erfindungsgemäßer elektrochemischer Gassensor neben einem Gehäuse und einem Elektrolyten eine Arbeitselektrode, welche aus reduziertem Graphenoxid als Elektrodenmaterial gebildet wird, eine Gegenelektrode, welche aus Kohlenstoffnanoröhren als Elektrodenmaterial gebildet wird. Insbesondere bevorzugt sind die Kohlenstoffnanoröhren in dieser Ausgestaltung in einem Verbundmaterial umfassend Glasfasern und Bariumcarbonat, welches als Absorptionsmittel eingesetzt wird, angeordnet. In dieser Ausgestaltung ist ein mit Borsäure geträgertes Vlies als Separatormaterial zwischen der Arbeits- und Gegenelektrode angeordnet.

In einer weiteren Ausgestaltung weist der erfindungsgemäße elektrochemische Gassensor zusätzlich noch eine Referenzelektrode und/oder eine Schutzelektrode auf, bevorzugt weist der Gassensor sowohl eine Schutzelektrode als auch eine Referenzelektrode auf. Eine Referenzelektrode (Bezugselektrode oder Vergleichselektrode) ist eine Elektrode mit einem konstanten Gleichgewichtspotential, das sich schnell und reproduzierbar einstellt. Eine Referenzelektrode wird als Bezugspunkt für die Messung von relativen Potentialen anderer Elektroden eingesetzt. Eine Schutzelektrode verhindert, dass z.B. Störstoffe aus dem Elektrolytraum an die Arbeitselektrode diffundieren, was zu Fehlsignalen an der Arbeitselektrode führen kann. Störstoffe können beispielsweise Verunreinigungen sein, welche an der Arbeitselektrode reduziert werden können. Die Schutzelektrode arbeitet üblicherweise reduktiv. Die einzelnen im Gassensor angeordneten Elektroden sind üblicherweise mittels Trennschichten (sog. Separatoren = ionendurchlässige Membranen) voneinander angeordnet.

Der Erfinder hat überraschenderweise gefunden, dass bei gleichzeitigem Zusatz einer Borverbindung und eines Absorptionsmittels die Dauerbegasungsfestigkeit und damit die Langzeitsignalstabilität erheblich verbessert werden konnte. Der erfindungsgemäße elektrochemische Gassensor weist insbesondere bei der Konzentrationsbestimmung saurer Gase, d. h. von Gasen und/oder Dämpfen welche in wässriger Lösung einen pH-Wert von kleiner 7 aufweisen, eine wesentlich verbesserte Langzeitsignalstabilität gegenüber den aus dem Stand der Technik bekannten Gassensoren auf, insbesondere bei der Konzentrationsbestimmung halogenhaltiger Gase, vor allem bei HF und über einen Zeitraum von wenigstens 12 bis 18 Monaten.

Ein weiterer Gegenstand der vorliegenden Erfindung ist ein Verfahren zur Konzentrationsbestimmung eines sauren Analytgases bereitzustellen, mit welchem über einen langen Zeitraum eine hohe Langzeitsignalstabilität gewährleistet wird. Dazu wird ein elektrochemischer Gassensor mit einem sauren Analytgas beaufschlagt, wobei das Analytgas durch eine Öffnung zumindest teilweise zur Arbeitselektrode diffundiert und an dieser elektrochemisch reduziert wird. Die dabei entstehenden Reaktionsprodukte (Ionen) wandern zumindest teilweise zur Gegenelektrode, wo diese zumindest teilweise wieder zu einem Oxidationsprodukt oxidiert werden. Das in den elektrochemischen Gassensor diffundierte Analytgas reagiert zumindest teilweise chemisch mit einer Borverbindung, insbesondere Borsäure und/oder Bortrioxid, welche vorzugsweise auf eine Trennschicht (Vlies) geträgert zwischen der Arbeitselektrode und der Gegenelektrode angeordnet ist. Das Oxidationsprodukt wird wenigstens teilweise von einem Absorptionsmittel absorbiert bzw. chemisch gebunden. Vorzugsweise ist das Absorptionsmittel an der Gegenelektrode angeordnet.

Der erfindungsgemäße elektrochemische Gassensor kann zur Konzentrationsbestimmung von sauren Gasen und/oder sauren Gasgemischen bzw. sauren Dämpfen eingesetzt werden.

Die Vorteile des erfindungsgemäßen elektrochemischen Gassensors sowie dessen möglicher Aufbau wird im nachfolgenden anhand der folgenden Figuren dargestellt. Es zeigen:
- **Fig. 1**: einen Vergleich der Messsignale eines erfindungsgemäßen Gassensors gegenüber einem aus dem Stand der Technik bekannten Gassensor bei Beaufschlagung mit HF als Analytgas; sowie
- **Fig. 2** und **Fig. 3**: einen alternativen Aufbau eines erfindungsgemäßen elektrochemischen Gassensors mit im Wesentlichen um 90° zueinander gedrehten Sensoraufbauten.

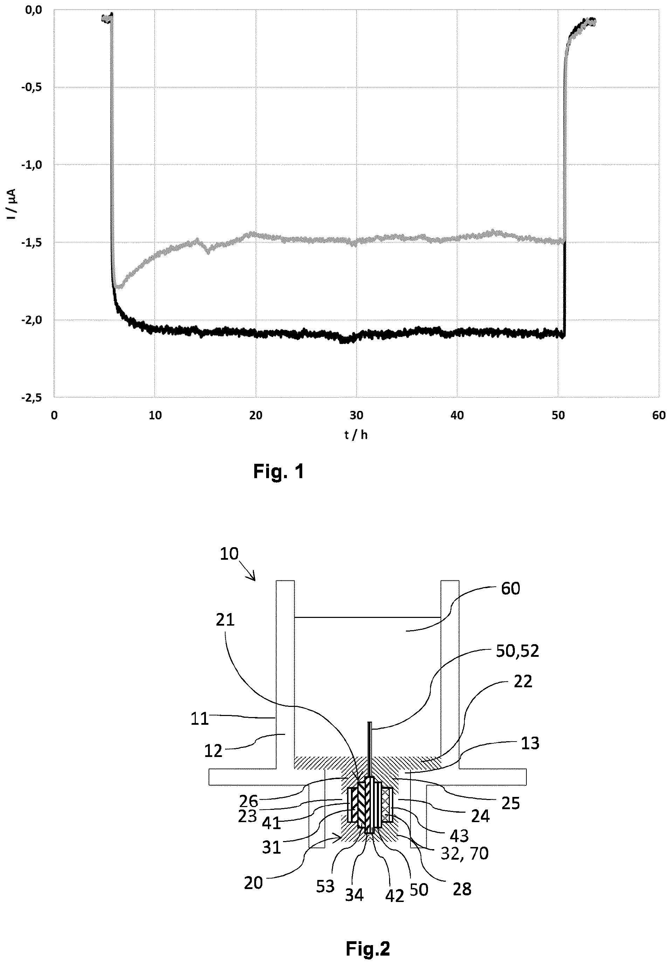

**Fig. 1** zeigt einen Vergleich der Messsignale zwischen einem erfindungsgemäßen elektrochemischen Gassensor (schwarze Linie) mit einem aus dem Stand der Technik bekannten elektrochemischen Gassensor (graue Linie) unter identischen Versuchsaufbauten. Aufgetragen ist die Stromstärke in Mikroampere über die Zeit in Stunden.

Der erfindungsgemäße elektrochemische Gassensor weist zusätzlich eine mit Borsäure geträgerte Trennschicht (mit Borsäure geträgertes Polymervlies) zwischen der Arbeitselektrode und der Gegenelektrode auf. Beide Gassensoren wurden zunächst mit Luft und anschließend mit 4,4 ppm Fluorwasserstoff als Analytgas für 45 Stunden beaufschlagt. Das obere, nicht erfindungsgemäße Signal gibt das Messsignal eines aus dem Stand der Technik bekannten Gassensors wieder, wohingegen das untere Signal das Messsignal des erfindungsgemäßen elektrochemischen Gassensors wiedergibt. Es ist deutlich zu erkennen, dass die graue Linie des aus dem Stand der Technik bekannten Gassensors bereits nach kurzer Begasungszeit bei einer Stromstärke von in etwa 1,75 µA (Mikroampere) auf ca. 1,5 µA "einbricht" und das Messsignal anschließend mit leichten Auf- und Abwärtsbewegungen des Messsignals bis zum Ende der Begasung nach 45 Stunden relativ konstant bei 1,5 µA verbleibt. Die schwarze Linie des erfindungsgemäßen Gassensors hingegen zeigt keinen vergleichbaren Einbruch der Stromstärke. Vielmehr bleibt das Messsignal des erfindungsgemäßen elektrochemischen Gassensors über den gesamten Messzeitraum hinweg stabil bei ca. zwei Mikroampere, und es zeigen sich insbesondere keine "Einbrüche" des Messsignals wie beim nicht-erfindungsgemäßen Gassensor.

Der in **Fig. 2** dargestellte erfindungsgemäße Gassensor 10 umfasst ein Gehäuse 11 und ein Elektrolytreservoir 12 für einen Elektrolyten 60 welches einen Reaktionsraum 21 umschließt. Mit dem Befestigungsabschnitt 22 ist der Elektrodenträger 20 in einer Aufnahme 13 des Elektrolytreservoirs 12 festgelegt. Der Reaktionsraum 21 wird von einem ersten Wandabschnitt 25 und einem zweiten Wandabschnitt 26 des Elektrodenträgers 20 begrenzt. Der Elektrodenträger 20 hat eine innere Oberfläche und eine äußere Oberfläche 28. In dem Gehäuse 11 sind eine erste Ausnehmung 23 und eine zweite Ausnehmung 24 ausgebildet. Die erste Ausnehmung 23 ist ein Gaseinlass, durch welchen das Analytgas in den Reaktionsraum 21 diffundieren kann. Die zweite Ausnehmung 24 ist ein Gasauslass, durch welchen an der Gegenelektrode 32 entstehendes Gas aus dem Reaktionsraum 21 ausdiffundieren kann. In dem Reaktionsraum 21 sind die Arbeitselektrode 31 und Gegenelektrode 32 des Gassensors 10 angeordnet. Zwischen der Arbeitselektrode 31 und der Gegenelektrode 32 befindet sich eine hydrophile, elektrolytdurchlässige Trennschicht 50. Die Arbeitselektrode 31 ist zur ersten Ausnehmung 23 von einer hydrophoben Membran 41 abgedeckt. Zwischen der Arbeitselektrode 31 und der Schutzelektrode 34 ist die Trennschicht 53 angeordnet, welche Borsäure enthält. Die Schutzelektrode 34 ist entlang des Diffusionsweges von der ersten Ausnehmung 23 zur zweiten Ausnehmung 24 durch die Membran 42 und die hydrophile, elektrolytdurchlässige Trennschicht 50 von der Gegenelektrode 32 getrennt. Die Gegenelektrode 32 ist mit dem Absorptionsmittel 70 geträgert und ist in Richtung der zweiten Ausnehmung 24 von der hydrophoben Membran 43 abgedeckt. In einer weiteren, nicht abgebildeten Ausgestaltung ist zusätzlich im Reaktionsraum 21 eine Referenzelektrode 33 angeordnet.

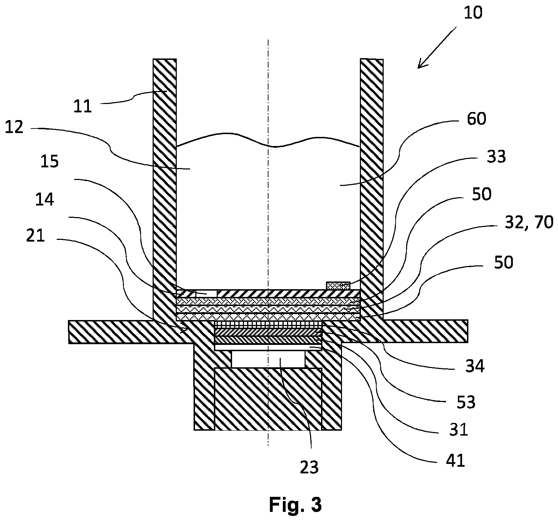

Bei dem in **Fig. 3** dargestellten Gassensor 10 ist im Gegensatz zu dem im Aufbau in Fig. 2 gezeigten Gassensor die Anordnung der Elektroden 31, 32 und die erste Ausnehmung 23 sowie der zwischen den Elektroden befindlichen Trennschichten und die Elektroden 31, 32 bedeckenden Membranen um 90° gedreht. Weiter ist im Gehäuse 11 eine Referenzelektrode 33 angeordnet. Durch die erste Ausnehmung 23 und die Membran 41 gelangt das Analytgas zur Arbeitselektrode 31. Hinter der Arbeitselektrode 31 ist die Borsäure enthaltende Trennschicht 53 angeordnet. In Richtung des Diffusionsweges von der ersten Ausnehmung 23 in Richtung der elektrolytdurchlässigen Öffnung 15, welche in einer elektrolytundurchlässigen Trennschicht 14 angeordnet ist, folgt nach der Borsäure enthaltenen Trennschicht 53 die Schutzelektrode 34 danach die von den Trennschichten 50 umgebene, mit Absorptionsmittel 70 geträgerte Gegenelektrode 32.

**Bezugszeichenliste:**

| | | | |
|---|---|---|---|
| 10 | Gassensor | 28 | Oberfläche |
| 11 | Gehäuse | 31 | Arbeitselektrode |
| 12 | Elektrolytreservoir | 32 | Gegenelektrode |
| 13 | Aufnahme | 33 | Referenzelektrode |
| 14 | elektrolytundurchlässige Trennschicht | 34 | Schutzelektrode |
| | | 41 | hydrophobe Membran |
| 15 | elektrolytdurchlässige Öffnung | 42 | Membran |
| 20 | Elektrodenträger | 43 | hydrophobe Membran |
| 21 | Reaktionsraum | 50 | hydrophile, elektrolytdurchlässige Trennschicht |
| 22 | Befestigungsabschnitt | | |
| 23 | Ausnehmung | 52 | Abschnitt |
| 24 | Ausnehmung | 53 | Borsäure enthaltende Trennschicht |
| 25 | Wandabschnitt | 60 | Elektrolyt |
| 26 | Wandabschnitt | 70 | Absorptionsmittel |

## Patentansprüche

1. Elektrochemischer Gassensor (10) für ein Analytgas, welches in wässriger Lösung einen pH-Wert kleiner 7 aufweist, der Gassensor wenigstens umfassend:
a) ein Gehäuse (11), wobei innerhalb des Gehäuses weiter angeordnet sind:
b) eine Arbeitselektrode (31);
c) eine Gegenelektrode (32);
d) ein mit Arbeits- und die Gegenelektrode in leitendem Kontakt stehenden Elektrolyten (60);
e) ein Absorptionsmittel (70), welches dazu geeignet ist, ein an der Arbeitselektrode (31) und/oder Gegenelektrode (32) in einem anwendungsgemäßen Zustand entstehendes Reaktionsprodukt zu absorbieren;
**dadurch gekennzeichnet, dass** der Gassensor (10) innerhalb des Gehäuses (11) zumindest eine Borverbindung aufweist, welche dazu geeignet ist, mit dem Analytgas chemisch zu reagieren.

2. Gassensor nach Anspruch 1, wobei die Borverbindung Borsäure (H₃BO₃) und/oder Bortrioxid (B₂O₃) umfasst, vorzugsweise Borsäure und/oder Bortrioxid ist.

3. Gassensor nach einem der vorhergehenden Ansprüche, wobei die Borverbindung in Pulverform und/oder auf ein Vlies geträgert und/oder als Borosilikatglas eingesetzt wird.

4. Gassensor nach einem der vorhergehenden Ansprüche, wobei die Borverbindung auf der Oberfläche der Arbeitselektrode (31) oder auf der Oberfläche der Gegenelektrode (32) oder zwischen Arbeitselektrode (31) und Gegenelektrode (32) angeordnet ist, bevorzugt zwischen Arbeitselektrode (31) und Gegenelektrode (32).

5. Gassensor nach einem der vorhergehenden Ansprüche, wobei das Analytgas halogenhaltig ist und/oder H₂S und/oder Essigsäure, insbesondere HF und/oder HCl und/oder HBr und/oder HI, besonders bevorzugt HF.

6. Gassensor nach einem der vorhergehenden Ansprüche, wobei das Absorptionsmittel zumindest teilweise an der Arbeitselektrode (31) und/oder der Gegenelektrode (32) angeordnet ist, vorzugsweise an der Gegenelektrode (32).

7. Gassensor nach einem der vorherigen Ansprüche, wobei das Absorptionsmittel eine Carbonatverbindung, bevorzugt eine Alkalicarbonatverbindung oder eine Erdalkalicarbonatverbindung, weiter bevorzugt BaCO₃ und/oder CaCO₃, insbesondere bevorzugt BaCO₃ umfasst oder ist.

8. Gassensor nach einem der vorhergehenden Ansprüche, wobei die Arbeitselektrode (31) ein Elektrodenmaterial umfasst ausgewählt aus der Gruppe:
Kohlenstoffnanoröhren, Graphen, diamantähnlicher Kohlenstoff, reduziertes Graphenoxid, bevorzugt reduziertes Graphenoxid.

9. Gassensor nach einem der vorhergehenden Ansprüche, wobei die Gegenelektrode (32) ein Elektrodenmaterial umfasst ausgewählt aus der Gruppe:
Kohlenstofffasern, Graphen, Glaskohlenstoff, Kohlenstoffnanoröhren, bevorzugt Kohlenstoffnanoröhren.

10. Gassensor nach einem der vorhergehenden Ansprüche, zusätzlich umfassend eine Referenzelektrode (33) und/oder eine Schutzelektrode (34).

11. Gassensor nach einem der vorherigen Ansprüche, wobei der Elektrolyt (60) ein organisches Lösungsmittel und ein Leitsalz umfasst, bevorzugt eine Zusammensetzung, die ein organisches Lösungsmittel, welches ein chinoides System enthält, und ein Leitsalz, welches ein organisches Kation aufweist, enthält.

12. Verfahren zur Konzentrationsbestimmung eines Analytgases, wobei das Analytgas in wässriger Lösung einen pH-Wert kleiner 7 aufweist, wobei das Verfahren wenigstens folgende Schritte umfasst:
a) Beaufschlagen eines elektrochemischen Gassensors (10) nach einem der Ansprüche 1 bis 11 mit dem Analytgas;
b) zumindest teilweise Reduktion des Analytgases an der Arbeitselektrode (31), wobei wenigstens ein Reaktionsprodukt zur Gegenelektrode (32) migriert;
c) zumindest teilweise Oxidation des Reaktionsprodukts an der Gegenelektrode (32) zu wenigstens einem Oxidationsprodukt;
d) zumindest teilweise chemische Reaktion des Analytgases mit einer Borverbindung;
e) zumindest teilweise Absorption des Oxidationsprodukts durch ein Absorptionsmittel.

13. Verwendung eines elektrochemischen Gassensors (10) nach einem der Ansprüche 1 bis 11, zur Konzentrationsbestimmung von sauren Gasen und/oder saure Gase enthaltenden Gasgemischen, insbesondere von HF.
